# DEMANDE DE BREVET EUROPEEN

(11) **EP 0 555 154 A1**
(43) Date de publication de la demande: **11.08.1993**
(21) Numéro de dépôt: 93400290.8
(22) Date de dépôt: 05.02.1993
(51) Int. Cl.: A61F 5/02

(54) **Ceinture de maintien lombaire**

(30) Priorité: 07.02.1992 FR 9201402
(71) Demandeur: Lelli, Charles, F-75019 Paris (FR); Lelli, Marc Louis Georges, F-75018 Paris (FR)
(72) Inventeur: Lelli, Charles, F-75019 Paris (FR); Lelli, Marc Louis Georges, F-75018 Paris (FR)
(74) Mandataire: Lemoine, Robert

(57) **Abrégé**

La ceinture de maintien lombaire selon l'invention comprend une pièce postérieure de maintien lombaire (1), une pièce antérieure de maintien abdominal (2) constituée de deux parties indépendantes (2a,2b), deux pièces latérales de maintien des hanches (3), des moyens de réglage en longueur (4,5) reliant les pièces latérales (3) à la pièce postérieure (1) et aux deux parties (2a,2b) de la pièce antérieure (2), et des moyens de fermeture (6) aptes à relier ces deux parties.

## Description

La présente invention concerne une ceinture de maintien lombaire.

Les ceintures actuelles de maintien lombaire sont en général constituées d'une simple sangle élastique comportant des barrettes métalliques dans sa partie médiane et des systèmes de fermeture au voisinage de ses extrémités libres.

Ces ceintures ne donnent toutefois pas pleinement satisfaction. Elles assurent en effet un maintien lombaire peu satisfaisant, les barrettes métalliques exerçant sur la colonne vertébrale de l'utilisateur une pression variant avec la posture et les mouvements de ce dernier. En outre, elles n'exercent pas de maintien abdominal efficace et équilibré. Par ailleurs, comme leurs parties terminales sont l'une rigide et l'autre élastique, elles ont tendance à tourner autour du corps de l'utilisateur, ce qui est gênant pour ce dernier.

La présente invention se propose plus particulièrement de remédier à ces inconvénients et, pour ce faire, elle a pour objet une ceinture de maintien lombaire qui se caractérise en ce qu'elle comprend une pièce postérieure de maintien lombaire, une pièce antérieure de maintien abdominal constituée de deux parties indépendantes, deux pièces latérales indépendantes de maintien des hanches, des moyens de réglage en longueur reliant les pièces latérales à la pièce postérieure et aux deux parties de la pièce antérieure, et des moyens de fermeture aptes à relier ces deux parties.

Lorsque cette ceinture est en place, sa pièce postérieure reste constamment en appui contre la colonne vertébrale de l'utilisateur et assure un maintien lombaire efficace quels que soient la posture et les mouvements de ce dernier.

Sa pièce antérieure maintient quant à elle convenablement la partie abdominale de l'utilisateur, ce qui n'est pas assuré par les ceintures actuelles.

Par ailleurs, les moyens de réglage en longueur permettent d'adapter facilement la ceinture à la taille de l'utilisateur de façon à ce que celui-ci ne soit pas gêné dans ses mouvements.

La ceinture conforme à l'invention a en outre la particularité de ne pas tourner autour du corps de l'utilisateur lorsque celui-ci se déplace ou effectue des mouvements variés.

Avantageusement, la pièce postérieure de maintien lombaire comprend une armature métallique comportant un cadre principal destiné à être placé le long de la colonne vertébrale de l'utilisateur et deux cadres secondaires fixés sur le cadre principal, perpendiculairement à celui-ci, ainsi qu'une enveloppe de protection renfermant l'armature métallique.

Grâce à la structure particulière de sa partie postérieure, la ceinture conforme à l'invention maintient fermement la partie lombaire de l'utilisateur qui peut ainsi exercer en toute sécurité des activités professionnelles ou sportives à risque.

De préférence, la face des côtés longitudinaux du cadre principal qui est destinée à être dirigée vers la colonne vertébrale est convexe tandis que la face correspondante des côtés longitudinaux des cadres secondaires présente trois tronçons concaves séparés par les côtés longitudinaux du cadre principal.

La forme particulière des cadres constituant l'armature métallique permet à la partie postérieure de la ceinture de se déformer élastiquement suivant le principe de l'arbalète pour s'adapter en permanence à la morphologie de l'utilisateur. Il convient par ailleurs de noter que les tronçons concaves centraux évitent que l'armature vienne au contact des apophyses épineuses postérieures de l'utilisateur.

Afin de faciliter l'application de la pièce postérieure de la ceinture contre la partie lombaire de l'utilisateur, il est souhaitable que l'enveloppe de protection soit réalisée en matière synthétique et présente deux échancrures latérales au niveau de ses parties situées entre les cadres secondaires, à l'extérieur du cadre principal.

Par ailleurs, pour éviter que la ceinture puisse transmettre à l'utilisateur les vibrations qui s'exerceraient sur elle, l'enveloppe de protection comprend un panneau interne en mousse basse densité solidaire d'un panneau externe en mousse haute densité pourvu d'un revêtement de protection, le panneau interne étant destiné à être dirigé vers la colonne vertébrale.

Selon un mode de réalisation particulier, les moyens de réglage en longueur comprennent des sangles fixées sur chacune des pièces latérales et passant dans des boucles prévues respectivement sur la pièce postérieure et sur les parties constitutives de la pièce antérieure, des organes de fixation complémentaires prévus respectivement sur les pièces latérales et sur les sangles étant destinés à être mis sélectivement en prise pour fixer les extrémités li- i-bres de ces dernières sur lesdites parties latérales dans des positions différentes.

Il suffit ainsi de mettre en prise des organes de fixation complémentaires particuliers pour ajuster la longueur de la ceinture et régler au mieux la tension de cette dernière en fonction de la taille de l'utilisateur.

Les organes de fixation complémentaires prévus sur les pièces latérales et sur les sangles passant dans les boucles de la pièce postérieure peuvent être des boutons-pression.

Quant aux organes de fixation complémentaires Prévus sur les pièces latérales et sur les sangles passant dans les boucles des parties constitutives de la pièce antérieure, ils peuvent être des rubans autoagrippants.

Afin d'augmenter le confort de l'utilisateur, il est préférable que les pièces latérales soient en tissu élastique.

Si l'utilisateur souhaite limiter l'allongement élastique des pièces latérales, par exemple pour accroître l'efficacité mécanique de la pièce postérieure, il est souhaitable que les sangles passant dans les boucles des parties constitutives de la pièce antérieure se prolongent au-dessus des sangles passant dans les boudes de la pièce postérieure, des rubans autoagrippants étant fixés sur les face en regard des sangles pour relier ces dernières entre elles.

Pour que l'utilisateur puisse exercer ses activités dans les meilleures conditions, il est par ailleurs préférable que la pièce antérieure soit plus petite que la pièce postérieure, et que ses parties constitutives comportent au moins une couche interne en mousse basse densité solidaire d'une couche externe en mousse haute densité pourvue d'un revêtement de protection, la couche interne étant destinée à être dirigée vers l'abdomen du patient.

D'autre part, pour éviter que la ceinture puisse se détacher intempestivement pendant que l'utilisateur exerce ses activités habituelles, il est souhaitable qu'elle comprenne deux moyens de fermeture constitués chacun d'une pièce mâle et d'une pièce femelle, les pièces mâles étant fixées sur l'une des parties constitutives de la pièce antérieure, tandis que les pièces femelles sont fixées sur l'autre partie.

Un mode d'exécution de la présente invention sera décrit ci-après à titre d'exemple nullement limitatif en référence aux dessins annexés dans lesquels :
- la figure 1 est une vue en perspective schématique d'une ceinture conforme à l'invention, la face externe de la ceinture étant tournée vers l'extérieur ;
- la figure 2 est une autre vue en perspective schématique de la ceinture visible sur la figure 1, sa face externe étant tournée vers l'intérieur ; et
- la figure 3 est une vue en plan avec arrachement partiel de la pièce postérieure de la ceinture.

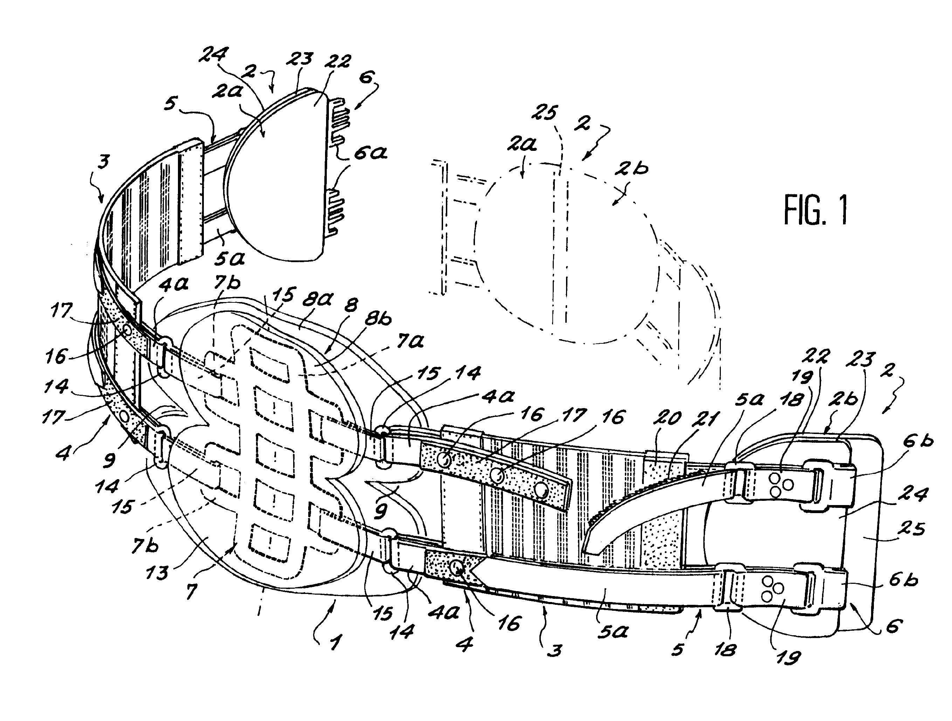

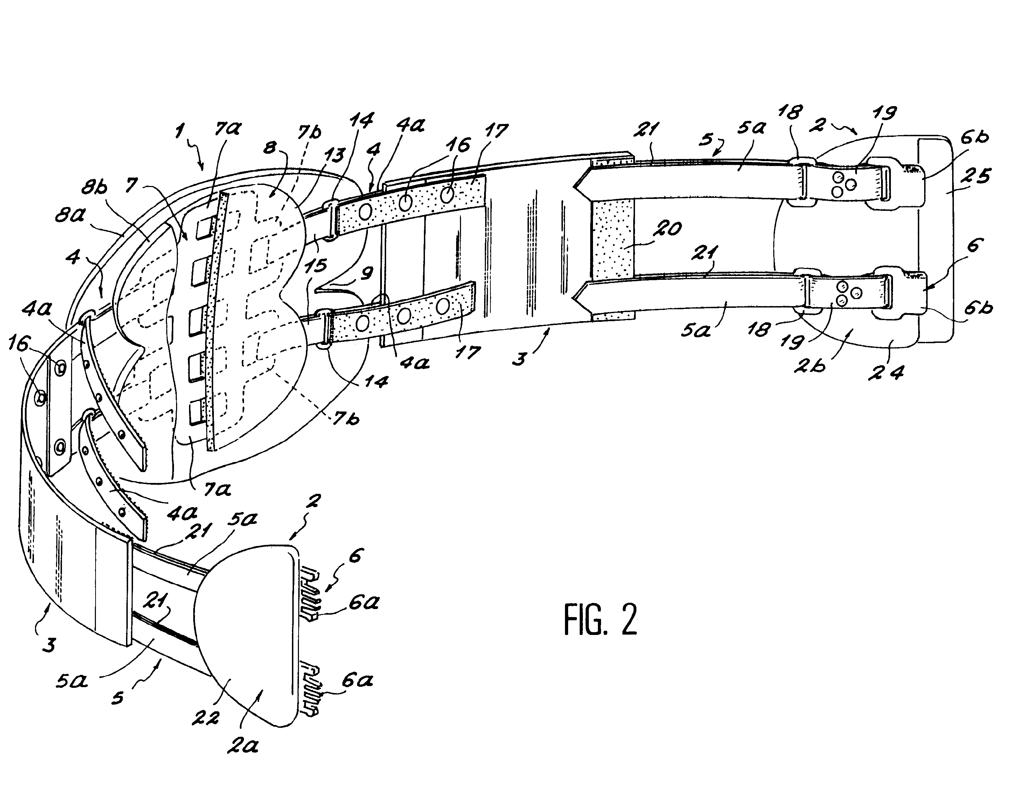

La ceinture de maintien lombaire que l'on peut voir sur les figures 1 et 2 comprend une pièce postérieure 1, une pièce antérieure 2 constituée de deux parties indépendantes 2a,2b (représentées en traits mixtes sur la figure 1, reliées l'une à l'autre), deux pièces latérales 3, des moyens de réglage postérieurs 4 reliant les pièces latérales 3 à la pièce postérieure 1, des moyens de réglage antérieurs 5 reliant quant à eux les pièces latérales 3 aux parties 2a,2b de la pièce antérieure 2, et deux moyens de fermeture 6 conçus pour relier les parties 2a,2b l'une à l'autre et fermer la ceinture.

La pièce postérieure 1 comprend une armature métallique 7 comportant un cadre principal 7a destiné à être placé le long de la colonne vertébrale de l'utilisateur, et deux cadres secondaires 7b fixés sur le cadre principal 7a, perpendiculairement à celui-ci, ainsi qu'une enveloppe de protection 8 renfermant l'armature 7.

La face des côtés longitudinaux du cadre principal 7a qui est située du côté de la face interne de la ceinture est convexe tandis que la face correspondante des côtés longitudinaux des cadres secondaires 7b présente trois tronçons concaves séparés par les côtés longitudinaux du cadre principal.

L'armature métallique 7 est déformable élastiquement et, grâce à la configuration particulière des cadres qui la constituent, elle permet à la partie postérieure 1 de la ceinture de rester en permanence en contact avec la région lombaire de l'utilisateur, quels que soient les mouvements de celui-ci.

L'enveloppe de protection 8 présente deux échancrures latérales 9 au niveau de ses parties situées entre les cadres secondaires 7b, à l'extérieur du cadre principal 7a, ces échancrures facilitant l'adaptation de la pièce postérieure 1 à la morphologie de l'utilisateur.

L'enveloppe 8, qui est réalisée en matière synthétique pour rendre la ceinture particulièrement confortable, comprend un panneau interne 8a situé du côté de la face interne de la ceinture et un panneau externe 8b situé du côté opposé, ces panneaux étant fixés l'un contre l'autre et enserrant l'armature métallique 7 entre eux.

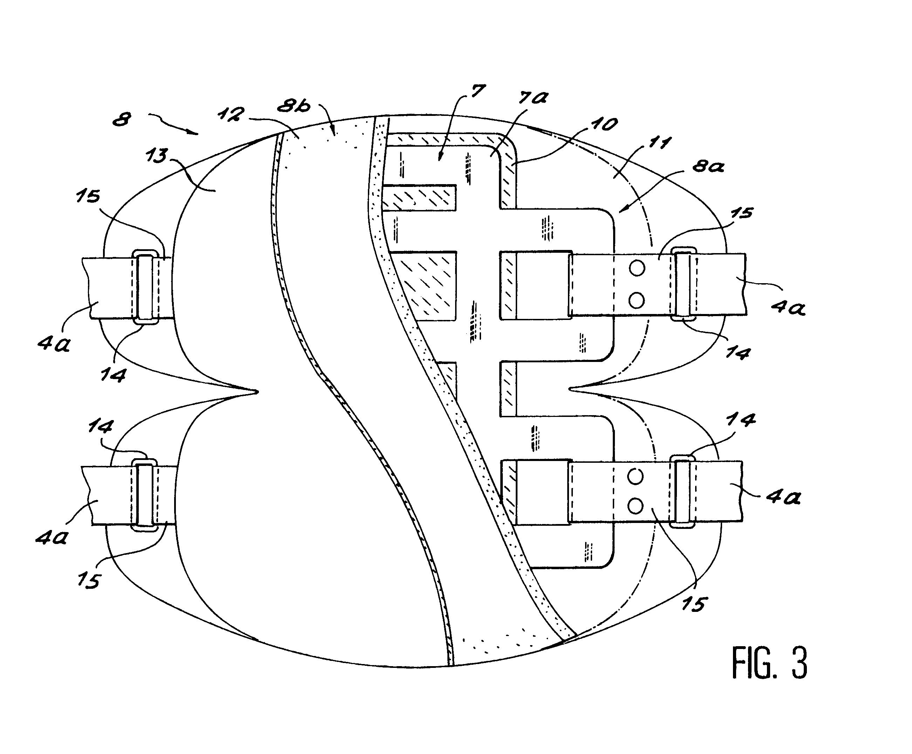

Dans l'exemple de réalisation envisagé ici, le panneau interne 8a est plus grand que le panneau externe 8b. Comme le montre la figure 3, il comprend une première plaquette de mousse basse densité 10 fixée contre le cadre principal 7a et ayant des dimensions légèrement supérieures à celles de ce dernier, et une seconde plaquette de mousse basse densité 11 fixée sur la plaquette 10, la plaquette 11 ayant des dimensions supérieures à celles de l'armature métallique 7.

Quant au panneau externe 8b, il comprend une première plaquette de mousse haute densité 12 fixée contre l'armature métallique et ayant des dimensions supérieures à celles de cette dernière, et une plaquette de revêtement de protection 13, par exemple en matériau imitant le cuir, recouvrant la plaque 11.

On notera ici que les différentes plaquettes constituant l'enveloppe 8 sont collées les unes aux autres et réalisées de préférence en mousse de polyéthylène, la plaquette en mousse haute densité constituant une protection externe en cas de choc et permettant de filtrer les vibrations tandis que les deux plaquettes en mousse basse densité sont destinées à protéger directement la colonne lombaire, à filtrer les vibrations, à éviter les refroidissements et à garantir un confort d'utilisation.

Bien entendu, l'enveloppe 8 pourrait avoir une structure différente de celle qui vient d'être décrite, sans que l'on sorte pour autant du cadre de la présente invention.

Les moyens de réglage postérieurs 4 comprennent quatre sangles 4a fixées deux par deux sur les pièces latérales 3, ces sangles faisant saillie sur les côtés transversaux des pièces latérales 3 qui sont tournés vers la pièce postérieure 1 et passant dans des boucles 14 portées par des attaches 15 fixées sur les côtés transversaux des cadres secondaires 7b de l'armature 7. Des boutons-pression 16 dont les éléments complémentaires sont fixés les uns sur la face externe des pièces latérales 3 et les autres sur la face des sangles 4a qui est destinée à être rabattue sur lesdites pièces latérales permettent de régler la distance entre ces dernières et la pièce postérieure 1. Il suffit en effet de mettre sélectivement en prise des boutons-pression particuliers 16 pour effectuer ce réglage.

On notera ici que les sangles 4a sont de préférence réalisées en textile synthétique, par exemple en fibres de polypropylène. Elles pourraient cependant être réalisées en tout autre matériau souple.

On notera également que leur face qui est tournée vers l'extérieur après leur rabattement contre les pièces latérales 3 porte, au voisinage de leur extrémité libre, un ruban autoagrippant 17 dont la fonction sera précisée ci-après.

Les moyens de réglage antérieurs 5 comprennent quatre sangles 5a fixées deux par deux sur les pièces latérales 3, ces sangles faisant saillie sur les côtés des pièces latérales 3 qui sont tournées vers les parties 2a,2b de la pièce antérieure 2 et passant dans des boucles 18 portées par des attaches 19 fixées sur la face externe des parties 2a,2b.

Les pièces latérales 3 sont pourvues le long de leur côté transversal qui est tourné vers les parties 2a,2b d'un ruban autoagrippant 20. De même, les sangles 5a sont pourvues sur leurface destinée à être rabattue sur les pièces latérales 3 d'un ruban autoagrippant 21.

On conçoit aisément qu'en appliquant des zones particulières des rubans 21 des sangles 5a contre les rubans 20 des pièces latérales 3, on peut régler de façon relativement précise la distance entre ces dernières et les parties 2a,2b de la pièce antérieure 2.

On précisera ici que les sangles 5a, comme les sangles 4a, sont de préférence réalisées en textile synthétique, par exemple en polypropylène.

En se référant plus particulièrement à la figure 1, on remarquera qu'il peut être souhaitable que les sangles antérieures 5a soient suffisamment longues pour que leur ruban autoagrippant 21 puisse être appliqué contre le ruban autoagrippant 17 des sangles postérieures 4a. En effet, comme les pièces latérales 3 sont de préférence réalisées en tissu élastique, la mise en prise des rubans autoagrippants 17 et 21 peut limiter de façon importante les possibilités d'extension des pièces latérales 3, ce qui peut être recherché par l'utilisateur lorsque celui-ci doit effectuer des mouvements de forte amplitude ou fournir des efforts importants.

En revanche, si l'utilisateur souhaite bénéficier des avantages procurés par l'élasticité des pièces latérales 3, il doit effectuer une fixation relâchée des sangles 5a.

La pièce antérieure 2 est plus petite que la pièce postérieure 1. Dans le mode de réalisation envisagé ici, ses parties constitutives 2a,2b sont constituées par la superposition d'une plaquette de mousse basse densité 22 située du côté de la face interne de la ceinture, d'une plaquette de mousse haute densité 23 et d'une plaquette de revêtement de protection 24, par exemple imitant le cuir, située du côté de la face externe de la ceinture.

Les plaquettes constituant les parties 2a,2b de la pièce antérieure 2 sont collées entre elles et réalisées de préférence en mousse de polyéthylène. Rien ne s'oppose cependant à ce qu'elles soient réalisées en un autre matériau ou à ce que leur nombre soit différent de 3.

On remarquera ici que la pièce 2b porte sur sa face située du côté interne de la ceinture une languette 25 destinée à venir sous la pièce 2a lorsque la ceinture est fermée, comme représenté en traits mixtes sur la figure 1.

Les attaches 19 sont fixées sur les parties 2a,2b de la pièce antérieure 2 par des rivets et portent les deux moyens de fermeture 6 au niveau de leur extrémité opposée à celle qui porte une boucle 18.

D'une manière connue en soi, les moyens de fermeture 6 sont constitués chacun d'une pièce mâle 6a fixée sur la partie 2a et d'une pièce femelle 6b fixée sur la partie 2b. Il suffit donc d'introduire les pièces mâles 6a dans les pièces femelles 6b pour fermer la ceinture et d'agir sur un ergot de verrouillage (non représenté) prévu sur les pièces mâles 6a pour séparer ces dernières des pièces femelles 6b et ouvrir la ceinture.

Pour installer la ceinture conforme à l'invention autour de son corps, l'utilisateur peut procéder comme suite :
- il pose à plat devant lui la ceinture de telle sorte que la face externe de celle-ci soit tournée vers lui ;
- il règle la distance entre la pièce postérieure 1 et les pièces latérales 3 en mettant en prise certains boutons-pression 16 dont les éléments complémentaires sont respectivement prévus sur les sangles 4a et sur les pièces latérales 3 ;
- il desserre les rubans autoagrippants 21 prévus sur les sangles 5a ;
- il revêt la ceinture en saisissant les parties 2a,2b de la pièce antérieure 2 et en veillant à ce que la pièce postérieure 1 vienne correctement en face de sa colonne vertébrale ;
- il met en prise les pièces mâles 6a et les pièces femelles 6b des moyens de fermeture 6 ;
- il règle la distance entre les pièces latérales 3 et les parties 2a,2b de la pièce antérieure 2 en appliquant contre les rubans autoagrippants 20 des pièces latérales 3 les parties appropriées des rubans autoagrippants 21 des sangles 5a. Pour une question de facilité, il est préférable qu'il effectue le réglage en commençant par les sangles 5a les plus basses ; et
- il fixe éventuellement les extrémités libres des sangles 5a sur les rubans autoagrippants 17 des sangles postérieures 4a.

Bien entendu, pour retirer la ceinture, l'utilisateur doit simplement séparer les pièces mâles et femelles des moyens de fermeture.

## Revendications

1. Ceinture de maintien lombaire comprenant une pièce postérieure de maintien lombaire (1) et une pièce antérieure de maintien abdominal (2) constituée de deux parties indépendantes (2a,2b) pourvues de moyens de fermeture (6) aptes à les relier l'une à l'autre, caractérisée en ce qu'elle comporte en outre deux pièces latérales indépendantes de maintien des hanches (3) reliées chacune à la pièce postérieure (1) et à l'une des deux parties (2a,2b) de la partie antérieure (2) par des moyens de réglage en longueur (4,5).

2. Ceinture selon la revendication 1, caractérisée en ce que la pièce postérieure de maintien lombaire (1) comprend une armature métallique (7) comportant un cadre principal (7a) destiné à être placé le long de la colonne vertébrale de l'utilisateur et deux cadres secondaires (7b) fixés sur le cadre principal (7a) perpendiculairement à celui-ci, ainsi qu'une enveloppe de protection (8) renfermant l'armature métallique (7).

3. Ceinture selon la revendication 2, caractérisée en ce que la la face des côtés longitudinaux du cadre principal (7a) qui est destinée à être dirigée vers la colonne vertébrale est convexe tandis que la face correspondante des côtés longitudinaux des cadres secondaires (7b) présente trois tronçons concaves séparés par les côtés longitudinaux du cadre principal (7a).

4. Ceinture selon la revendication 2, caractérisée en ce que l'enveloppe de protection (8) est réalisée en matière synthétique et présente deux échancrures latérales (9) au niveau de ses parties situées entre les cadres secondaires, à l'extérieur du cadre principal (7a).

5. Ceinture selon la revendication 4, caractérisée en ce que l'enveloppe de protection (8) comprend un panneau interne (8a) en mousse basse densité solidaire d'un panneau externe (8b) en mousse haute densité pourvu d'un revêtement de protection (13), le panneau interne (8a) étant destiné à être dirigé vers la colonne vertébrale.

6. Ceinture selon l'une quelconque des revendications précédentes, caractérisée en ce que les moyens de réglage en longueur (4,5) comprennent des sangles (4a,5a) fixées sur chacune des pièces latérales (3) et passant dans des boucles (14,18) prévues respectivement sur la pièce postérieure (1) et sur les parties constitutives (2a,2b) de la pièce antérieure (2), des organes de fixation complémentaires (16 ; 20,21) prévus respectivement sur les pièces latérales (3) et sur les sangles (4a,5a) étant destinés à être mis sélectivement en prise pour fixer les extrémités libres de ces dernières sur lesdites parties latérales dans des positions différentes.

7. Ceinture selon la revendication 6, caractérisée en ce que les organes de fixation complémentaires (16) prévus sur les pièces latérales (3) et sur les sangles (4a) passant dans les boucles (14) de la pièce postérieure (1) sont des boutons-pression.

8. Ceinture selon la revendication 7, caractérisée en ce que les organes de fixation complémentaires (20,21) prévus sur les pièces latérales (3) et sur les sangles (5a) passant dans les boucles (18) des parties constitutives (2a,2b) de la pièce antérieure (2) sont des rubans autoagrippants.

9. Ceinture selon l'une quelconque des revendications précédentes, caractérisée en ce que les pièces latérales (3) sont en tissu élastique.

10. Ceinture selon l'une quelconque des revendications 6 à 9, caractérisée en ce que les sangles (5a) passant dans les boucles (18) des parties constitutives (2a,2b) de la pièce antérieure (2) se prolongent au-dessus des sangles (4a) passant dans les boucles (14) de la pièce postérieure (1), des rubans autoagrippants (17,21) étant fixés sur les face en regard des sangles (4a,5a) pour relier ces dernières entre elles.

11. Ceinture selon l'une quelconque des revendications précédentes, caractérisée en ce que la pièce antérieure (2) est plus petite que la pièce postérieure (1).

12. Ceinture selon l'une quelconque des revendications précédentes, caractérisée en ce que les parties constitutives (2a,2b) de la pièce antérieure (2) comportent au moins une couche interne en mousse basse densité (22) solidaire d'une couche externe en mousse haute densité (23) pourvue d'un revêtement de protection (24), la couche interne étant destinée à être dirigée vers l'abdomen du patient.

13. Ceinture selon l'une quelconque des revendications précédentes, caractérisée en ce qu'elle comprend deux moyens de fermeture (6) constitués chacun d'une pièce mâle (6a) et d'une pièce femelle (6b), les pièces mâles (6a) étant fixées sur l'une (2a) des parties constitutives de la pièce antérieure (2a), tandis que les pièces femelles (6b) sont fixées sur l'autre partie (2b).
